(19) Europäisches Patentamt
European Patent Office
Office européen des brevets

(11) EP 0 998 913 A1

(12) EUROPÄISCHE PATENTANMELDUNG

(43) Veröffentlichungstag:
**10.05.2000 Patentblatt 2000/19**

(51) Int. Cl.[7]: **A61K 7/48**

(21) Anmeldenummer: **99117909.4**

(22) Anmeldetag: **13.09.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **26.09.1998 DE 19844261**

(71) Anmelder:
**Beiersdorf Aktiengesellschaft**
**20245 Hamburg (DE)**

(72) Erfinder:
- **Bleckmann, Andreas**
  **22926 Ahrensburg (DE)**
- **Schneider, Günther, Dr.**
  **22607 Hamburg (DE)**
- **Röckl, Manfred**
  **22880 Wedel (DE)**
- **Nielsen, Jens**
  **24558 Henstedt-Ulzburg (DE)**

# (54) Kosmetische und dermatologische Zubereitungen in Form von W/O-Emulsionen mit einem Gehalt an niedermolekularen Siliconen

(57) Kosmetische oder dermatologische Zubereitungen, welche

(a) Wasser-in-Öl-Emulsionen darstellen und
(b) einen Gehalt von 3 bis zu 30 Gew.-%, bevorzugt 5 - 10 Gew.-% eines oder mehrerer Silicone der allgemeinen Struktur

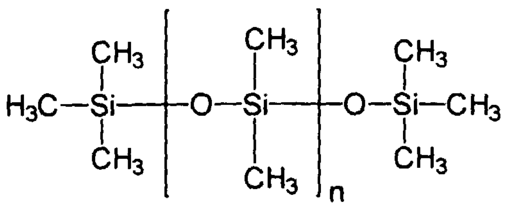

,

bezogen auf das Gesamtgewicht der Zubereitung, aufweisen, wobei n Zahlen zwischen 5 und 50 annehmen kann,
(c) höchstens 1 Gew.-% an cyclischen Siliconen aufweisen, bezogen auf das Gesamtgewicht der Zubereitung, wobei bevorzugt wird, vollständig auf solche Öle zu verzichten.
(d) höchstens 10 Gew.-%, bevorzugt 0,5 - 3 Gew.-% an Siliconen der allgemeinen Struktur

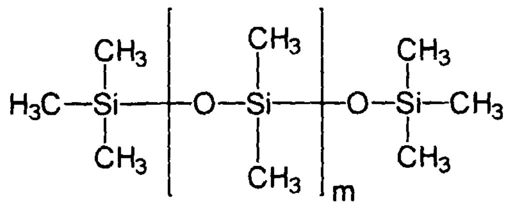

aufweisen, bezogen auf das Gesamtgewicht der Zubereitung, wobei m Zahlen größer als 50 annimmt.

EP 0 998 913 A1

## Beschreibung

Die vorliegende Erfindung betrifft kosmetische und dermatologische Zubereitungen in Form von W/O-Emulsionen mit einem Gehalt an niedermolekularen Siliconen.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letzlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Epidermis ist ein stratifiziertes Gewebe, in dem die äußere Schicht, die Hornschicht (Stratum corneum), den für die Barrierefunktion bedeutenden Teil darstellt. Das heute in der Fachwelt anerkannte Hautmodell von Elias (*P. M. Elias, Structure and Function of the Stratum Corneum Permeability Barrier, Drug Dev. Res.* ***13****, 1988, 97-105*) beschreibt die Hornschicht als Zwei-Komponenten-System, ähnlich einer Ziegelsteinmauer (Ziegelstein-Mörtel-Modell). In diesem Modell entsprechen die Hornzellen (Korneozyten) den Ziegelsteinen, die komplex zusammengesetzte Lipidmembran in den Interzellularräumen entspricht dem Mörtel. Dieses System stellt im wesentlichen eine physikalische Barriere gegen hydrophile Substanzen dar, kann aber aufgrund seiner engen und mehrschichtigen Struktur gleichermaßen auch von lipophilen Substanzen nur schwer passiert werden.

Die vorliegende Erfindung betrifft in einer besonderen Ausführungsform kosmetische oder pharmazeutische Zubereitungen mit vermindertem Klebrigkeitsgefühl, Verfahren zu ihrer Herstellung sowie die Verwendung von Wirkstoffen zur Herabminderung des Klebrigkeitsgefühles kosmetischer Zubereitungen.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Häufige Erscheinungsformen kosmetischer oder dermatologischer Zubereitungen sind feindisperse Mehrphasensysteme, in welchen eine oder mehrere Fett- bzw. Ölphasen neben einer bzw. mehreren Wasserphasen vorliegen. Von diesen Systemen sind wiederum die eigentlichen Emulsionen die am weitesten verbreiteten.

Weiterhin war es eine Aufgabe der Erfindung, kosmetische Grundlagen für kosmetische Zubereitungen zu entwickeln, die sich durch gute Hautverträglichkeit auszeichnen.

Ferner war eine Aufgabe der vorliegenden Erfindung, Produkte mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen, aber auch ausgesprochen medizinisch-pharmazeutische Darreichungsformen geschaffen werden, zum Beispiel Zubereitungen gegen Akne und andere Hauterscheinungen.

Die Formulierung stabiler Wasser-in-Öl-Emulsionen mit einer überdurchschnittlichen Hautpflegeleistung (Hautbefeuchtung sowie Hautglättung) in Kombination mit sehr guten sensorischen Eigenschaften gestaltet sich derzeit als nach wie vor sehr schwierig. Dies ist nicht zuletzt darin begründet, daß der Zusatz hautpflegewirksamer Additive - wie z.B. Moisturizer, bestimmte Lipide oder Filmbildner - zu instabilen Emulsionen und/oder einem stumpfen bzw. klebrigen Hautgefühl beim Verteilen des Produktes auf der Haut führt, so daß die Emulsionen dann zwar möglicherweise wirksam, aber gleichzeitig sensorisch unakzeptabel und/oder instabil sind.

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Es hat sich in den Vergangenheit durchaus bewährt, zumindest Teile einer kosmetisch verwendeten Ölphase aus der Gruppe der cyclischen und/oder linearen Silicone zu wählen, welche im Rahmen der vorliegenden

Offenbarung auch als „Siliconöle" bezeichnet werden. Solche Silicone oder Siliconöle können als Monomere vorliegen, welche in der Regel durch Strukturelemente charakterisiert sind, wie folgt:

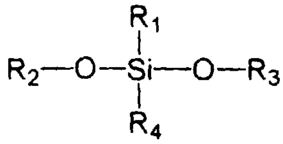

Als linearen Silicone mit mehreren Siloxyleinheiten werden im allgemeinen durch Strukturelemente charakterisiert wie folgt:

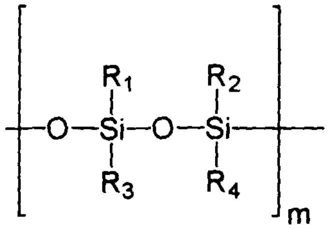

,

wobei die Siliciumatome mit gleichen oder unterschiedlichen Alkylresten und/oder Arylresten substituiert werden können, welche hier verallgemeinernd durch die Reste $R_1$ - $R_4$ dargestellt sind (will sagen, daß die Anzahl der unterschiedlichen Reste nicht notwendig auf bis zu 4 beschränkt ist). m kann dabei Werte von 2 - 200.000 annehmen.

Auch cyclische Silicone werden als Bestandteile von kosmetischen Ölphasen eingesetzt, wobei sie im allgemeinen durch Strukturelemente charakterisiert werden, wie folgt:

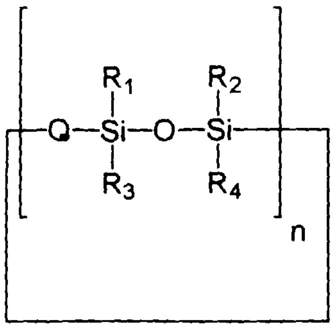

,

und wobei die Siliciumatome mit gleichen oder unterschiedlichen Alkylresten und/oder Arylresten substituiert werden können, welche hier verallgemeinernd durch die Reste $R_1$ - $R_4$ dargestellt sind. n kann dabei in der Regel Werte von 3/2 bis 6 annehmen. Gebrochene Werte für n berücksichtigen, daß ungeradzahlige Anzahlen von Siloxylgruppen im Cyclus vorhanden sein können.

Oftmals wird Phenyltrimethicon als Siliconöl gewählt. Auch andere Silikonöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methylphenylsiloxan), Cetyldimethicon, Behenoxydimethicon sind gängige Einsatzstoffe.

Cyclische Silicone haben jedoch einige Nachteile, hauptsächlich formulierungstechnischer Natur. Gleichwohl galten sie bisher als unverzichtbar, wollte man kosmetische Zubereitungen auf der Grundlage von Siliconölen formulieren.

Natürlich ist dem Fachmann eine Vielzahl von Möglichkeiten bekannt, stabile W/O-Zubereitungen zur kosmetischen oder dermatologischen Anwendung zu formulieren, beispielsweise in Form von Cremes und Salben, die im Bereich von Raum- bis Hauttemperatur streichfähig sind, oder als Lotionen und Milche, die in diesem Temperaturbereich eher fließfähig sind. Der Stand der Technik kennt allerdings nur wenige Formulierungen, die so dünnflüssig sind, daß sie beispielsweise sprühbar wären.

Unter dem Begriff „Viskosität" versteht man die Eigenschaft einer Flüssigkeit, der gegenseitigen laminaren

Verschiebung zweier benachbarter Schichten einen Widerstand (Zähigkeit, innere Reibung) entgegenzusetzen. Man definiert heute diese sogenannte dynamische Viskosität nach $\eta=\tau/D$ als das Verhältnis der Schubspannung zum Geschwindigkeitsgradienten senkrecht zur Strömungsrichtung. Für newtonsche Flüssigkeiten ist $\eta$ bei gegebener Temperatur eine Stoffkonstante mit der SI-Einheit Pascalsekunde (Pa • s).

Der Quotient $\nu=\eta/\rho$ aus der dynamischen Viskosität $\eta$ und der Dichte $\rho$ der Flüssigkeit wird als kinematische Viskosität $\nu$ bezeichnet und in der SI-Einheit $m^2/s$ angegeben.

Als Fluidität ($\varphi$) bezeichnet man den Kehrwert der Viskosität ($\varphi=1/\eta$). Bei Salben und dergleichen wird der Gebrauchswert unter anderem mitbestimmt von der sogenannten Zügigkeit. Unter der Zügigkeit einer Salbe oder Salbengrundlage oder dergleichen versteht man deren Eigenschaft, beim Abstechen verschieden lange Fäden zu ziehen; dementsprechend unterscheidet man kurz- und langzügige Stoffe.

Während die graphische Darstellung des Fließverhaltens Newtonscher Flüssigkeiten bei gegebener Temperatur eine Gerade ergibt, zeigen sich bei den sogenannten nichtnewtonschen Flüssigkeiten in Abhängigkeit vom jeweiligen Geschwindigkeitsgefälle D (Schergeschwindigkeit $\dot{\gamma}$) bzw. der Schubspannung $\tau$ oft erhebliche Abweichungen. In diesen Fällen läßt sich die sogenannte scheinbare Viskosität bestimmen, die zwar nicht der Newtonschen Gleichung gehorcht, aus der sich jedoch durch graphische Verfahren die wahren Viskositätswerte ermitteln lassen.

Die Fallkörperviskosimetrie ist lediglich zur Untersuchung newtonscher Flüssigkeiten sowie von Gasen geeignet. Sie basiert auf dem Stokes-Gesetz, nach dem für das Fallen einer Kugel durch eine sie umströmende Flüssigkeit die dynamische Viskosität $\eta$ aus

$$\eta = \frac{2r^2(\rho_K - \rho_{Fl}) \cdot g}{9 \cdot v}$$

bestimmbar ist, wobei

> r = Radius der Kugel, v = Fallgeschwindigkeit, $r_K$ = Dichte der Kugel, $r_{Fl}$ = Dichte der Flüssigkeit und g = Fallbeschleunigung.

W/O-Emulsionen mit einer geringen Viskosität, die eine Lagerstabilität aufweisen, wie sie für marktgängige Produkte gefordert wird, sind nach dem Stand der Technik nur sehr aufwendig zu formulieren. Dementsprechend ist das Angebot an derartigen Formulierungen äußerst gering. Gleichwohl könnten derartige Formulierungen dem Verbraucher bisher nicht gekannte kosmetische Leistungen bieten.

W/O-Emulsionen mit einer höheren Viskosität sind durchaus gängige Zubereitungen. Unter Verzicht auf cyclische Silikonöle neigen solche Zubereitungen allerdings dazu, kosmetisch anspruchslos zu wirken und sich durch wenig ansprechendes Hautgefühl auszuzeichnen.

Eine Aufgabe der vorliegenden Erfindung war es, Grundlagen für Zubereitungen zur Verfügung zu stellen, welche es einesteils erlauben, höherviskose Zubereitungen zu erstellen (welche man landläufig als Crèmes bezeichnen würde), aber auch andererseits eine gute Grundlage für Zubereitungen darstellen, die eine geringe oder gar sehr geringe Viskosität aufweisen (also beispielsweise Formulierungen, welche man landläufig als Lotionen bezeichnen würde), ohne aber von einigen der Nachteile des Standes der Technik behaftet zu sein.

Die EP-B-0 577 817 beschreibt beispielsweise stabile Wasser-in-Öl-Emulsionen für den kosmetischen oder pharmazeutischen Gebrauch, dadurch gekennzeichnet, daß sie eine Fettphase in einem Mengenverhältnis von 15 zu 40 Gew.-%, das aus mindestens einem Silicon in einem Mengenverhältnis von 15 bis 40 Gew.-% besteht, jeweils in bezug auf das Gesamtgewicht der Emulsion, sowie eine wäßrige Phase mit einem Gehalt an mindestens einem gegenüber Elektrolyten unempfindlichen wäßrigen Geliermittel enthält, wobei der Emulgator in der Emulsion ein Alkyl- oder Alkoxydimethicon-Copolyol darstellt.

Solche Zubereitungen zeichnen sich jedoch stets durch einen Anteil an cyclischen Siliconen, und daher durch die besagten Nachteile aus. Solche Nachteile zu beseitigen war also Aufgabe der vorliegenden Erfindung.

Erstaunlicherweise werden diese Aufgaben gelöst durch kosmetische oder dermatologische Zubereitungen, welche

> (a) Wasser-in-Öl-Emulsionen darstellen und
> (b) einen Gehalt von 3 bis zu 30 Gew.-%, bevorzugt 5 - 10 Gew.-% eines oder mehrerer Silicone der allgemeinen Struktur

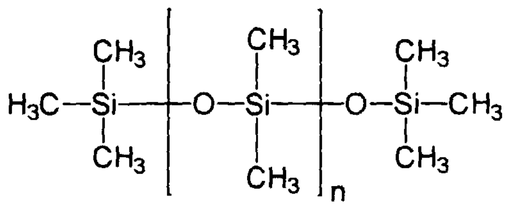

,

bezogen auf das Gesamtgewicht der Zubereitung, aufweisen, wobei n Zahlen zwischen 5 und 50 annehmen kann,

(c) höchstens 1 Gew.-% an cyclischen Siliconen aufweisen, bezogen auf das Gesamtgewicht der Zubereitung, wobei bevorzugt wird, vollständig auf solche Öle zu verzichten.

(d) höchstens 10 Gew.-%, bevorzugt 0,5 - 3 Gew.-% an Siliconen der allgemeinen Struktur

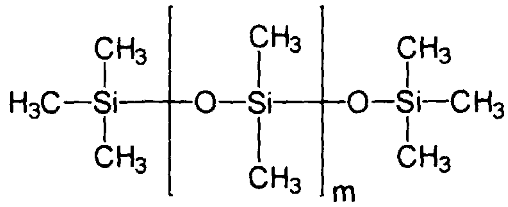

aufweisen, bezogen auf das Gesamtgewicht der Zubereitung, wobei m Zahlen größer als 50 annimmt.

Es war für den Fachmann daher nicht vorauszusehen gewesen, daß die erfindungsgemäßen Zubereitungen

- besser als feuchtigkeitsspendende Zubereitungen wirken,
- einfacher zu formulieren sein,
- besser die Hautglättung fördern,
- sich durch besser Pflegewirkung auszeichen,
- besser als Vehikel für kosmetische und medizinisch-dermatologische Wirkstoffe dienen
- bessere sensorische Eigenschaften, wie beispielsweise die Verteilbarkeit auf der Haut oder das Einzugsvermögen in die Haut, aufweisen würden
- höhere Stabilität gegenüber Zerfall in Öl- und Wasserphasen aufweisen und
- sich durch bessere Bioverträglichkeit auszeichnen würden

als die Zubereitungen des Standes der Technik.

Die erfindungsgemäßen Zubereitungen sind sowohl fließfähig als auch cremeartig formulierbar, besitzen sehr gute kosmetische Eigenschaften, insbesondere was die Klebrigkeit betrifft, und weisen eine sehr gute Hautverträglichkeit sowie Hautpflegeleistung auf.

Es ist von Vorteil, den Durchschnittswert n in dem oder den Siliconen der allgemeinen Struktur unter Punkt (b) aus dem Bereich von 5 - 20, bevorzugt 12 -16 zu wählen.

Als ein besonders vorteilhafter Vertreter solcher Siliconöle hat sich das Siliconöl AK 10 der Gesellschaft Wacker-Chemie GmbH herausgestellt, dessen Durchschnittswert für n bei etwa 14 liegt.

Erfindungsgemäß ist es möglich und vorteilhaft, den Anteil der Ölphase der erfindungsgemäßen Zubereitungen im Bereich von 3 bis 30 Gew.-%, bezogen auf das Gesamtgewicht der Zubereitungen, frei zu wählen.

Als Grundbestandteile der erfindungsgemäßen Zubereitungen können verwendet werden:

- Wasser oder wäßrige Lösungen
- wäßrige ethanolische Lösungen
- natürliche Öle und/oder chemisch modifizierte natürliche Öle und/oder synthetische Öle;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet.

Die Ölphase der Emulsionen im Sinne der vorliegenden Erfindung besteht erfindungsgemäß vorzugsweise vollständig aus Siliconen der unter Punkt (b) aufgeführten Art, wobei es allerdings möglich ist, ohne große Nachteile in kauf zu nehmen, bis zur Hälfte des Gesamtgewichtes der Ölkomponenten aus der Gruppe anderer Ölkomponenten zu wählen. Diese können dann vorteilhaft gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse.

Weitere vorteilhafte Fett- und/oder Wachskomponenten sind chemisch modifzierte Wachse und synthetische Wachse, wie beispielsweise die unter den Handelsbezeichnungen Syncrowax HRC (Glyceryltribehenat), Syncrowax HGLC ($C_{16-36}$-Fettsäuretriglycerid) und Syncrowax AW 1C ($C_{18-36}$-Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder $C_{30-50}$-Alkyl Bienenwachs), Polyalkylenwachse, Polyethylenglykolwachse, aber auch chemisch modifizierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise $C_{20-40}$-Alkylstearat, $C_{20-40}$-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan.

Erfindungsgemäß können die Fett- und/oder Wachskomponenten sowohl einzeln als auch im Gemisch vorliegen.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkybenzoat und 2-Ethylhexylisostearat, Mischungen aus $C_{12-15}$-Alkybenzoat und Isotridecylisononanoat sowie Mischungen aus $C_{12-15}$-Alkybenzoat, 2-Ethylhexylisostearat und Isotridecylisononanoat.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden.

Erfindungsgemäße W/O-Emulsionen können vorteilhaft mit Hilfe der üblichen W/O-Emulgatoren, gewünschtenfalls unter Zuhilfenahme von O/W-Emulgatoren bzw. weiteren Coemulgatoren hergestellt werden.

W/O-Emulsionen entsprechend der vorliegenden Erfindung enthalten einen oder mehrere Emulgatoren, insbesondere vorteilhaft gewählt aus der Gruppe der folgenden Substanzen, die in der Regel als W/O-Emulgatoren wirken:

Sorbitanstearat, Sorbitanoleat,, Lecithin, Glyceryllanolat, Lanolin, mikrokristallines Wachs (Cera microcristallina) im Gemisch mit Paraffinöl (Paraffinum liquidum), Ozokerit, hydriertem Ricinusöl, Glycerylisostearat, Polyglyceryl-3-Oleat, Wollwachssäuregemische, Wollwachsalkoholgemische, Pentaerythrithylisostearat, Polyglyceryl-3

Diisostearat, Sorbitan Oleat im Gemisch mit hydriertem Ricinusöl, Bienenwachs (Cera alba) und Stearinsäure, Natriumdihydroxycetylphosphat im Gemisch mit Isopropylhydroxycetylether, Methylglucosedioleat, Methylglucosedioleat im Gemisch mit Hydroxystearat und Bienenwachs, Mineralöl im Gemisch mit Petrolatum und Ozokerit und Glyceryloleat und Lanolinalkohol, Petrolatum im Gemisch mit Ozokerit und hydriertem Ricinusöl und Glycerylisostearat und Polyglyceryl-3-oleat, PEG-7-hydriertes Ricinusöl, Sorbitanoleat im Gemisch mit PEG-2-hydriertem Ricinusöl, Ozokerit und hydriertem Ricinusöl, Sorbitanisostearat im Gemisch mit PEG-2-hydriertem Ricinusöl, Polyglyceryl-4-isostearat, Polyglyceryl-4-isostearat im Gemisch mit Cetyldimethiconcopolyol und Hexyllaurat, Laurylmethiconcopolyol, Cetyldimethiconcopolyol, Acrylat/ $C_{10\text{-}30}$-Alkylacrylat-Crosspolymer, Sorbitanisostearat, Poloxamer 101, Polyglyceryl-2-dipolyhydroxystearat, Polyglyceryl-3-Diisostearat, Polyglyceryl-4-dipolyhydroxystearat, PEG-30-dipolyhydroxystearat, Diisostearoylpolyglyceryl-3-diisostearat, Polyglyceryl-2-dipolyhydroxystearat, Polyglyceryl-3-dipolyhydroxystearat, Polyglyceryl-4-dipolyhydroxystearat, Polyglyceryl-3-dioleat.

W/O-Emulsionen entsprechend der vorliegenden Erfindung enthalten gewünschtenfalls einen oder mehrere Emulgatoren, insbesondere vorteilhaft gewählt aus der Gruppe der folgenden Substanzen, die in der Regel als O/W-Emulgatoren wirken:

Glycerylstearat im Gemisch mit Ceteareth-20, Ceteareth-25, Ceteareth-6 im Gemisch mit Stearylalcohol, Cetylstearylalcohol im Gemisch mit PEG-40-Ricinusöl und Natriumcetylstearylsulfat, Triceteareth-4 Phosphat, Glycerylstearat, Natriumcetylstearylsulfat, Lecithin Trilaureth-4 Phosphat, Laureth-4 Phosphat, Stearinsäure, Propylenglycolstearat SE, PEG-25-hydriertes Ricinusöl, PEG-54-hydriertes Ricinusöl, PEG-6 Caprylsäure/Caprinsäureglyceride, Glyceryloleat im Gemisch mit Propylenglycol, PEG-9-Stearat, Ceteth-2, Ceteth-20, Polysorbat 60, Glycerylstearat im Gemisch mit PEG-100 Stearat, Glycerylmyristat, Glyceryllaurat, PEG-40-Sorbitanperoleat, Laureth-4, Ceteareth-3, Isostearylglycerylether, Cetylstearylalkohol im Gemisch mit Natrium Cetylstearylsulfat, Laureth-23, Steareth-2, Glycerylstearat im Gemisch mit PEG-30 Stearat, PEG-40-Stearat, Glycol Distearat, PEG-22-Dodecyl Glycol Copolymer, Polyglyceryl-2-PEG-4-Stearat, Ceteareth-20, Methylglucosesesquistearat, Steareth-10, PEG-20-Stearat, Steareth-2 im Gemisch mit PEG-8 Distearat, Steareth-21, Steareth-20, Isosteareth-20, PEG-45/ Dodecylglycol-Copolymer, Methoxy-PEG-22/Dodecylglycol-Copolymer, PEG-40-Sorbitanperoleat, PEG-40-Sorbitanperisostearat, PEG-20-Glycerylstearat, PEG-20-Glycerylstearat, PEG-8-Bienenwachs, Polyglyceryl-2-laurat, Isostearyldiglycerylsuccinat, Stearamidopropyl-PG-dimoniumchloridphosphat, Glycerylstearat SE, Ceteth-20, Triethylcitrat, PEG-20-Methylglucosesesquistearat, Ceteareth-12, Glycerylstearatcitrat, Cetylphosphat, Sorbitansesquioleat, Triceteareth-4-Phosphat, Trilaureth-4-Phosphat, Polyglycerylmethylglucosedistearat, Kaliumcetylphosphat, Isosteareth-10, Polyglyceryl-2-sesquiisostearat, Ceteth-10, Oleth-20, Isoceteth-20, Glycerylstearat im Gemisch mit Ceteareth-20, Ceteareth-12, Cetylstearylalcohol und Cetylpalmitat, Cetylstearylalcohol im Gemisch mit PEG-20 Stearat, PEG-30-Stearat, PEG-40-Stearat, PEG-100-Stearat.

Es war insbesondere überraschend, daß erfindungsgemäße kosmetische oder dermatologische W/O-Emulsionen, welche sich durch einen Gehalt an Siliconemulgatoren auszeichnen, ganz besonders vorteilhafte Eigenschaften aufweisen. Derlei W/O-Emulsionen stellen demzufolge besonders bevorzugte Ausführungsformen der vorliegenden Erfindung dar.

Erfindungsgemäß können die Siliconemulgatoren vorteilhaft aus der Gruppe grenzflächenaktive Substanzen aus der Gruppe der Alkylmethiconcopolyole und/oder Alkyl-Dimethiconcopolyole gewählt werden, insbesondere aus der Gruppe der Verbindungen, welche gekennzeichnet sind durch die folgende chemische Struktur:

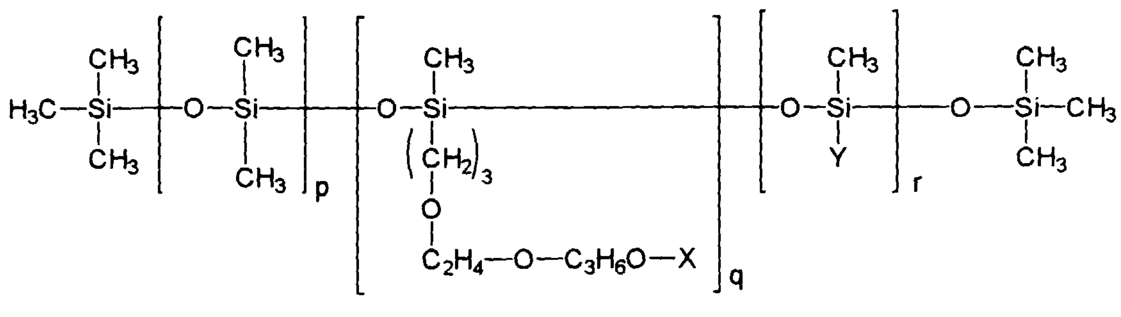

bei welcher X und Y unabhängig voneinander gewählt werden aus der Gruppe H sowie der verzweigten und unverzweigten Alkylgruppen, Acylgruppen und Alkoxygruppen mit 1 - 24 Kohlenstoffatomen, p eine Zahl von 0 - 200 darstellt, q eine Zahl von 1 - 40 darstellt, und r eine Zahl von 1 - 100 darstellt.

Ein Beispiel für besonders vorteilhaft im Sinne der vorliegenden Erfindung zu verwendende grenzflächenaktiven Substanzen ist das Cetyl Dimethiconcopolyol, welches von der Gesellschaft Th.Goldschmidt AG unter der Warenbezeichnung ABIL® EM 90 verkauft wird.

Ein weiteres Beispiel für besonders vorteilhaft im Sinne der vorliegenden Erfindung zu verwendende grenzflächenaktiven Substanzen ist das Cyclomethicon Dimethiconcopolyol, welches von der Gesellschaft Th.Goldschmidt AG unter der Warenbezeichnung ABIL® EM 97 verkauft wird.

Weiterhin hat sich als ganz besonders vorteilhaft der Emulgator Laurylmethiconcopolyol herausgestellt, welcher unter der Warenbezeichnung Dow Corning® 5200 Formulation Aid von der Gesellschaft Dow Corning Ltd. erhältlich ist.

Die Gesamtmenge an erfindungsgemäß verwendeten Siliconemulgatoren in den erfindungsgemäßen kosmetischen oder dermatologischen Zubereitungen wird vorteilhaft aus dem Bereich von 0,1 - 10,0 Gew.-%, bevorzugt 0,5 - 5,0 Gew.-% gewählt, bezogen auf das Gesamtgewicht der Zubereitungen.

Erfindungsgemäße Emulsionen im Sinne der vorliegenden Erfindung, z.B. in Form einer Hautschutzcrème, einer Hautlotion, einer kosmetischen Milch, beispielsweise in Form einer Sonnenschutzcreme oder einer Sonnenschutzmilch, sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Es ist ebenfalls von Vorteil, von den erfindungsgemäßen Eigenschaften in Form von dekorativen Kosmetika (Make-Up-Formulierungen) Gebrauch zu machen.

Günstig sind auch solche kosmetischen und dermatologischen Zubereitungen, die in der Form eines Sonnenschutzmittels vorliegen. Vorzugsweise enthalten diese neben dem erfindungsgemäß verwendeten Wirkstoff zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescrèmes gewöhnlich UV-A- bzw. UV-B-Filtersubstanzen eingearbeitet.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:

- 3-Benzylidencampher und dessen Derivate, z.B. 3-(4-Methylbenzyliden)campher,
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin

Als wasserlösliche Substanzen sind vorteilhaft:

- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;

- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Die erfindungsgemäßen kosmetischen und dermatologischen Zubereitungen können kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, organische Lösungsmittel oder Silikonderivate.

Es ist vorteilhaft, wenn die erfindungsgemäßen W/O-Emulsionen sich durch einen Anteil an Elektrolyten auszeichnen. Erfindungsgemäß vorteilhaft werden der oder die Elektrolyte gewählt aus der Gruppe

(1) der Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte sind vorteilhaft, z.B. Lactate, Acetate, Benzoate, Propionate, Tartrate, Citrate, Aminosäuren und deren Salze und andere mehr. Vergleichbare Effekte sind auch durch Ethylendiamintetraessigsäure und deren Salze zu erzielen.
Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Besonders bevorzugt sind Kaliumchlorid, Kochsalz, Magnesiumsulfat, Zinksulfat und Mischungen daraus.

Erfindungsgemäß werden der oder die Elektrolyte ferner vorteilhaft gewählt aus der Gruppe

(2) Bestimmte wasserlösliche, zumeist als Alkalisalze vorliegende wasserlösliche UV-Filtersubstanzen, insbesondere solche, die an ihrem Molekülgerüst eine oder mehrere Sulfonsäuregruppen bzw. Sulfonatgruppen tragen:
Die 2-Phenylbenzimidazol-5-sulfonsäure und ihre Salze, beispielsweise das Natrium-, Kalium- oder ihr Triethanolammonium-Salz

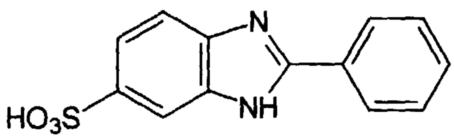

,

Sulfonsäure-Derivate von Benzophenonen, vorzugsweise 2-Hydroxy-4-methoxy-benzophenon-5-sulfonsäure und ihre Salze, beispielsweise das entsprechende Natrium-, Kalium- oder Triethanolammonium-Salz:

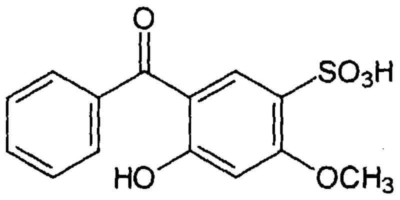

Sulfonsäure-Derivate des 3-Benzylidencamphers, wie z.B. 4-(2-Oxo-3-bornylidenmethyl)benzolsulfonsäure und ihre Salze, beispielsweise das entsprechende Natrium-, Kalium- oder Triethanolammonium-Salz:

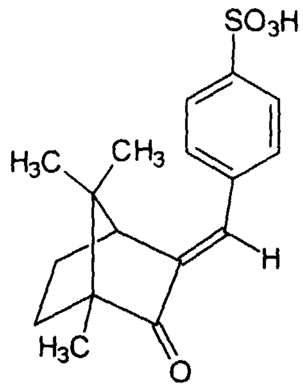

Die 2-Methyl-5-(2-oxo-3-bornylidenmethyl)benzolsulfonsäure und ihre Salze, beispielsweise das entsprechende Natrium-, Kalium- oder Triethanolammonium-Salz:

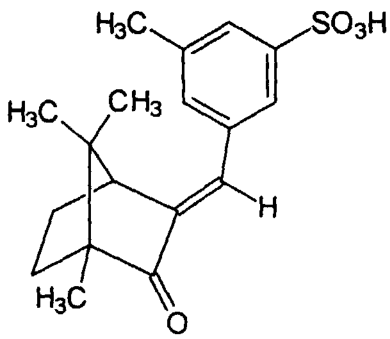

Das 1,4-di(2-oxo-10-Sulfo-3-bornylidenmethyl-) Benzol und dessen Salze (die entprehenden 10 Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet:

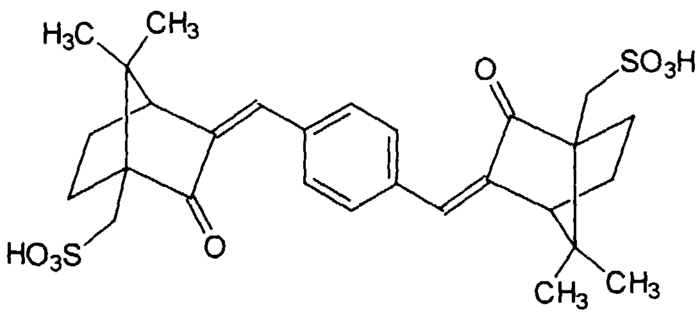

Erfindungsgemäß werden der oder die Elektrolyte weiterhin vorteilhaft gewählt aus der Gruppe

(3) der Aminosäuren und deren Salze bzw. deren Anionen. Aminosäuren sind Bestandteil des natürlichen Feuchtigkeitsfaktors (der sogenannte Natural Moisturizing Factor). Der Zusatz von Aminosäuren, insbesondere essentieller Aminosäuren, ist als vorteilhaft anzusehen, da über Hydratationsvorgänge Feuchtigkeit in der Haut gebunden werden kann.

Aminosäuren mit besonders vorteilhafter kosmetischer bzw. dermatologischer Wirkung sind Glycin, Alanin, Valin, Leucin, Isoleucin, Phenylalanin, Tyrosin, Prolin, Hydroxyprolin, Serin, Threonin, Cystein, Methionin, Tryptophan, Arginin.

Erfindungsgemäß werden der oder die Elektrolyte weiter vorteilhaft gewählt aus der Gruppe

(4) der kosmetisch und dermatologisch relevanten α-Hydroxycarbonsäuren, α-Ketocarbonsäuren und β-Hydroxycarbonsäuren und insbesondere deren Salze, wobei die Kationen vorteilhaft gewählt werden können aus der Gruppe Ammonium,- Alkylammonium,- Alkalimetall-, Erdalkalimetall,- Magnesium-, Eisen- bzw. Zinkionen.

α-Hydroxycarbonsäuren, welche kosmetisch oder dermatologisch relevant sind, folgen der allgemeinen Formel

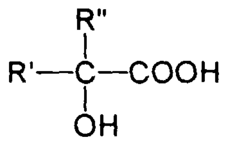

β-Hydroxycarbonsäuren, welche kosmetisch oder dermatologisch relevant sind, folgen der allgemeinen Formel

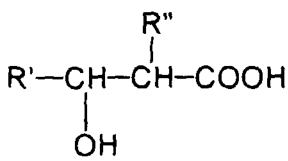

bzw.

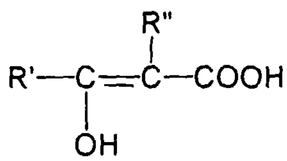

α-Ketocarbonsäuren, welche kosmetisch oder dermatologisch relevant sind, folgen der allgemeinen Formel

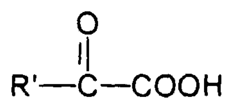

wobei jeweils R' und R'' unabhängig voneinander gewählt werden aus der Gruppe

(a1) H- ,
(a2) verzweigtes oder unverzweigtes $C_{1-25}$-Alkyl-,
(a3) mit einer oder mehreren Carboxylgruppen und/oder Hydroxygruppen und/oder Aldehydgruppen und/oder Oxogruppen (Ketogruppen) substituiertes verzweigtes oder unverzweigtes $C_{1-25}$-Alkyl-
(a4) Phenyl-,
(a5) mit einer oder mehreren Carboxylgruppen und/oder Hydroxygruppen und/oder verzweigten und/oder unverzweigten $C_{1-25}$-Alkylgruppen substituiertes Phenyl-,
oder wobei das α-Kohlenstoffatom und das β-Kohlenstoffatom der β-Hydroxycarbonsäure mit R' und R'' zusammen eine
(a6) unsubstituierte Cycloalkylgruppe oder Arylgruppe mit 3 bis 7 Ringatomen oder eine
(a7) mit einer oder mehreren Carboxylgruppen und/oder Hydroxygruppen und/oder Oxogruppen (Ketogruppen) und/oder verzweigten und/oder unverzweigten $C_{1-25}$-Alkylgruppen substituierte Cycloalkylgruppe oder Arylgruppe mit 3 bis 7 Ringatomen
ausbildet und
wobei die α-Hydroxycarbonsäuren oder die β-Hydroxycarbonsäuren oder die α-Ketocarbonsäuren gegebenenfalls in Form ihrer physiologisch verträglichen Salze vorliegen können.

Es folgen vorteilhaft im Rahmen der vorliegenden Erfindung zu verwendende α-Hydroxycarbonsäuren, β-Hydroxycarbonsäuren und α-Ketocarbonsäuren, wobei diese auch stellvertretend für ihre Salze bzw. Anionen aufgeführt werden:

Die Salicylsäure (auch 2-Hydroxybenzoësäure, Spirsäure), welche durch die Struktur

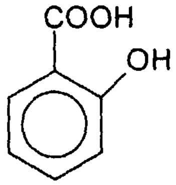

gekennzeichnet ist. Bekanntermaßen wirkt Salicylsäure antibakteriell und keratolytisch und ist Bestandteil mancher kosmetischen oder pharmazeutischen Zubereitungen.

Die den erfindungsgemäß verwendeten α-Hydroxycarbonsäuren werden vorteilhaft gewählt aus folgenden Substanzklassen:

(a2) α-Hydroxyfettsäuren, wobei diese wiederum besonders vorteilhaft aus der Gruppe der $C_{10-18}$-Alkylcarbonsäuren gewählt werden,
(a3) α-Hydroxyzuckersäuren, aliphatische α-Hydroxyfruchtsäuren,
(a4) unsubstituierte aromatische α-Hydroxycarbonsäuren (z.B. Mandelsäure) bzw.
(a5) substituierte aromatische α-Hydroxycarbonsäuren.

Die unter Punkt (a2) fallenden α-Hydroxyfettsäuren werden besonders vorteilhaft gewählt aus der Gruppe

- α-Hydroxycarbonsäuren, gemäß der Formel

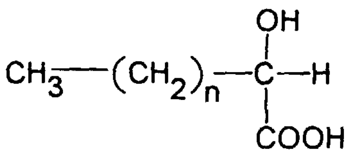

und/oder

- α-Hydroxy-isocarbonsäuren, gemäß der Formel

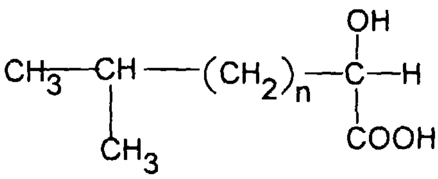

und/oder

- α-Hydroxy-anteisocarbonsäuren, gemäß der Formel

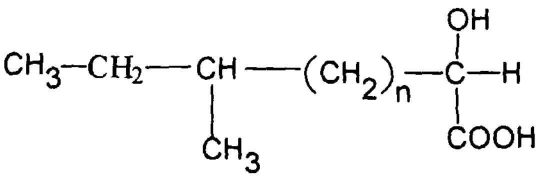

,

wobei n jeweils eine Zahl von 7 bis 31 darstellt.

Vorteilhaft ist weiter, Gemische solcher aliphatischen α-Hydroxycarbonsäuren, insbesondere in Form von Wollwachssäuregemischen zu verwenden, in welchen der Gehalt an α-Hydroxycarbonsäuren 20 - 30 Gew.-%, bezogen auf die Gesamtzusammensetzung beträgt.

Die unter Punkt (a3) fallenden α-Hydroxyzuckersäuren werden besonders vorteilhaft gewählt aus der Gruppe der

- Aldonsäuren, z.B. Gluconsäure, Galactonsäure
- Aldarsäuren, z.B. Glucarsäure, Galactarsäure (aber auch die Fruchtsäure Weinsäure, die ebenfalls unter die Definition der Aldarsäure fällt)
- Uronsäuren, z.B. Glucuronsäure, Galacturonsäure
- Glycerinsäure

Die unter Punkt (a3) fallenden aliphatischen α-Hydroxyfruchtsäuren werden besonders vorteilhaft gewählt aus der Gruppe Äpfelsäure, Milchsäure, Citronensäure, Weinsäure.

Äpfelsäure (Hydroxybernsteinsäure) ist durch folgende chemische Struktur gekennzeichnet:

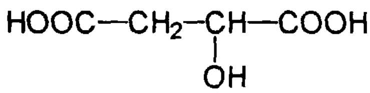

Milchsäure (2-Hydroxypropansäure) ist durch folgende chemische Struktur gekennzeichnet:

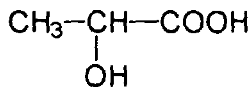

Citronensäure (2-Hydroxy-1,2,3-propantricarbonsäure) ist durch folgende chemische Struktur gekennzeichnet:

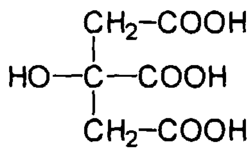

Bekanntermaßen wird Citronensäure zur Pufferung kosmetischer und/oder dermatologischer Zubereitungen, aber auch als Synergist für Antioxidantien in der Haut- und Haarkosmetik verwendet.

Weinsäure (Dihydroxybernsteinsäure) ist durch folgende chemische Struktur gekennzeichnet:

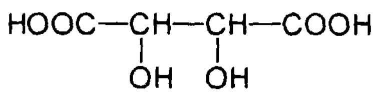

Bevorzugte α-Ketocarbonsäure ist die Brenztraubensäure (α-Oxopropansäure). Sie zeichnet sich durch folgende Struktur aus:

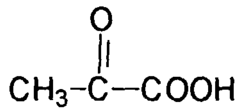

Die Höchstmenge der einzusetztenden Elektrolyte ist letztendlich abhängig von deren Löslichlichkeit in der wäßrigen Phase. Grundsetzlich setzt die erfindungsgemäße Lehre aber keine Höchstmengen als Schranke, da es gegebenenfalls ja sogar vorteilhaft sein mag, aus welchen Gründen auch immer, einen über die Löslichkeit eines Elektrolytes hinausgehende zusätzliche Menge dieses Elektrolytes, beispielsweise als ungelösten Festkörper, in eine kosmetische oder dermatologische Zubereitung einzuarbeiten.

Es ist ferner vorteilhaft, den Zubereitungen im Sinne der vorliegenden Erfindung weitere antiirritative oder antientzündliche Wirkstoffe zuzugeben, insbesondere Batylalkohol (α-Octadecylglycerylether), Selachylalkohol (α-9-Octadecenylglycerylether), Chimylalkohol (α-Hexadecylglycerylether), Bisabolol und/oder Panthenol.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, ψ-Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Zubereitungen gemäß der vorliegenden Erfindung können auch Verwendung als Grundlage für kosmetische oder dermatologische Desodorantien bzw. Antitranspirantien finden. Alle für Desodorantien bzw. Antitranspirantien gängigen Wirkstoffe können vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure.

Keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Wasserphase der kosmetischen Zubereitungen im Sinne der vorliegenden Erfindung kann auch Gelcharakter aufweisen, die neben einem wirksamen Gehalt am erfindungsgemäß eingesetzten Substanzen und dafür üblicherweise verwendeten Lösungsmitteln, bevorzugt Wasser, noch weitere organische Verdickungsmittel, z.B. Gummiarabikum, Xanthangummi, Natriumalginat, Stärke und Stärkederivate (z.B. Distärkephosphat), Cellulose, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise organisch modifizierte oder auch unmodifizierte Hectorite, Bentonite, oder dergleichen, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Ferner kann es von Vorteil sein, Zubereitungen gemäß der Erfindung grenz- bzw. oberflächenaktive Agentien zuzufügen, beispielsweise kationische Emulgatoren wie insbesondere quaternäre Tenside.

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Vorteilhaft ist auch, kationische Polymere (z.B. Jaguar C 162 [Hydroxypropyl Guar Hydroxypropyltrimonium Chloride] bzw. modifizierten Magnesiumaluminiumsilikaten (z.B. Quaternium-18-Hectorit, welches z. B. unter der Handelsbezeichnung Bentone® 38 bei der Firma Rheox erhältlich ist, oder Stearalkonium Hectorit, welches z. B. unter der Handelsbezeichnung Softisan® Gel bei der Hüls AG erhältlich ist), einzusetzen.

Erfindungsgemäße Zubereitungen können vorteilhaft auch Ölverdickungsmittel enthalten, um die taktilen Eigenschaften der Emulsion und die Stiftkonsistenz zu verbessern. Vorteilhafte Ölverdickungsmittel im Sinne der vorliegenden Erfindung sind beispielsweise weitere Feststoffe, wie z. B. hydrophobe Siliciumoxide des Typs Aerosil®, welche von der Degussa AG erhältlich sind. Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974 und/oder Aerosil® R976.

Ferner sind auch sogenannte Metallseifen (d. h. die Salze höherer Fettsäuren mit Ausnahme der Alkalisalze) vorteilhafte Ölverdickungsmittel im Sinne der vorliegenden Erfindung, wie beispielsweise Aluminium-Stearat, Zink-Stearat und/oder Magnesium-Stearat.

Ebenfalls vorteilhaft ist, Zubereitungen gemäß der Erfindung amphotere bzw. zwitterionischen Tensiden (z.B. Cocoamidopropylbetain) und Moisturizern (z.B. Betain) zuzusetzen. Vorteilhaft zu verwendende amphotere Tenside sind beispielsweise Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat, N-Alkylaminosäuren, beispielsweise Aminopropylalkylglutamid, Alkylaminopropionsäure, Natriumalkylimidodipropionat und Lauroamphocarboxyglycinat.

Die Menge der ober- bzw. grenzflächenaktiven Substanzen (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Erfindungsgemäße Zubereitungen können auch Wirkstoffe (eine oder mehrere Verbindungen) enthalten, welche gewählt werden aus der Gruppe: Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin $B_1$, das Vitamin $B_{12}$ das Vitamin $D_1$, aber auch Bisabolol, ungesättigte Fettsäuren, namentlich die essentiellen Fettsäuren (oft auch Vitamin F genannt), insbesondere die γ-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter. Vorteilhaft ist es auch, die Wirkstoffe aus der Gruppe der rückfettenden Substanzen zu wählen, beispielsweise Purcellinöl, Eucerit® und Neocerit®.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die nachfolgenden Beispiele sollen die vorliegende Erfindung verdeutlichen.

| **Beispiel 1 (W/O-Emulsion):** | |
|---|---|
| | Gew.-% |
| PEG-7-hydriertes Ricinusöl | 4,00 |
| Wollwachsalkohol | 1,50 |
| Bienenwachs | 3,00 |
| Vaseline | 4,00 |
| Ozokerit | 4,00 |
| Paraffinöl, subliquidum | 5,00 |
| Dimethicone Wacker AK 10 | 5,00 |
| Glycerin | 15,00 |
| Octyl Methoxycinnamat | 2,50 |
| Methylbenzylidene Camphor | 2,50 |
| Tocopherolacetat | 1,00 |
| Magnesiumsulfat 7 $H_2O$ | 0,70 |
| Parfüm, Konservierungsmittel, NaOH, Farbstoffe, Antioxidantien | q.s. |
| Wasser | ad 100,00 |

| **Beispiel 2 (W/O-Emulsion):** | |
|---|---|
| | Gew.-% |
| PEG-7-hydriertes Ricinusöl | 4,00 |
| Wollwachsalkohol | 1,50 |
| Bienenwachs | 3,00 |
| Vaseline | 9,00 |
| Ozokerit | 4,00 |
| Dimethicone Wacker AK 10 | 10,00 |
| Sorbit | 15,00 |
| Magnesiumsulfat 7 $H_2O$ | 0,70 |
| Parfüm, Konservierungsmittel, NaOH, Farbstoffe, Antioxidantien | q.s. |
| Wasser | ad 100,00 |

| **Beispiel 3 (W/O-Emulsion):** | |
|---|---|
| | Gew.-% |
| PEG-7-hydriertes Ricinusöl | 4,00 |
| Wollwachsalkohol | 1,50 |
| Bienenwachs | 3,00 |
| Vaseline | 9,00 |
| Ozokerit | 4,00 |
| Dimethicone Wacker AK 10 | 5,00 |
| Dimethicone Wacker AK 20 | 5,00 |
| Urea | 10,00 |
| Magnesiumsulfat 7 $H_2O$ | 0,70 |
| Milchsäure | 0,30 |
| Natriumlaktat | 2,50 |
| Parfüm, Konservierungsmittel, NaOH, Farbstoffe, Antioxidantien | q.s. |
| Wasser | ad 100,00 |

| **Beispiel 4 (W/O-Emulsion):** | |
|---|---|
| | Gew.-% |
| PEG-7-hydriertes Ricinusöl | 4,00 |
| Wollwachsalkohol | 1,50 |
| Bienenwachs | 3,00 |
| Vaseline | 9,00 |
| Ozokerit | 4,00 |
| Paraffinöl, subliquidum | 2,50 |
| Dimethicone Wacker AK 10 | 3,00 |
| Dimethicone Th. Goldschmidt ABIL 20 | 3,00 |
| Dimethicone Wacker AK 100 | 1,00 |
| Dimethicone Th. Goldschmidt ABIL 350 | 0,50 |
| Glycerin | 15,00 |
| Tocopherolacetat | 1,00 |
| Zinksulfat 7 $H_2O$ | 0,70 |
| Glycin | 1,50 |
| Tocopherolacetat | 0,50 |
| Parfüm, Konservierungsmittel, NaOH, Farbstoffe, Antioxidantien | q.s. |
| Wasser | ad 100,00 |

| **Beispiel 5 (W/O-Emulsion):** | |
|---|---|
| | Gew.-% |
| Polyglyceryl-3 Dioleat | 3,50 |
| Ozokerit | 3,00 |
| Bienenwachs | 2,00 |
| Paraffinöl, subliquidum | 10,00 |
| Dimethicone Dow Corning 200 Fluid, 5 cs | 5,00 |
| Dimethicone Wacker AK 10 | 2,50 |
| Dimethicone Wacker AK 20 | 2,00 |
| Cyclomethicone Dow Corning 345 Fluid | 0,50 |
| Serin | 0,50 |
| Sorbit | 9,00 |
| Glycerin | 9,00 |
| Magnesiumsulfat 7 $H_2O$ | 0,70 |
| Parfüm, Konservierungsmittel, NaOH, Farbstoffe, Antioxidantien | q.s. |
| Wasser | ad 100,00 |

| **Beispiel 6 (W/O-Emulsion):** | |
|---|---|
| | Gew.-% |
| Laurylmethicone Copolyol | 1,00 |
| Cetyldimethicone Copolyol | 1,50 |
| Paraffinöl, subliquidum | 7,00 |
| Dimethicone Wacker AK 10 | 5,00 |
| Dimethicone Th. Goldschmidt ABIL 20 | 1,00 |
| Dimethicone Wacker AK 100 | 1,00 |
| Dimethicone WackerAK 1000 | 0,50 |
| Cylomethicone DC 245 Fluid | 0,50 |
| Weizenkeimöl | 3,00 |
| Capric/Caprylic Triglyceride | 4,00 |
| Glycerin | 10,00 |
| Natriumchlorid | 1,00 |
| Parfüm, Konservierungsmittel, NaOH, Farbstoffe, Antioxidantien | q.s. |
| Wasser | ad 100,00 |

| Beispiel 7 (W/S-Emulsion): | |
|---|---|
| | Gew.-% |
| Cetyldimethicone Copolyol | 2,00 |
| Dimethicone Wacker AK 10 | 10,00 |
| Dimethicone Th. Goldschmidt ABIL 20 | 5,00 |
| Dimethicone Wacker AK 35 | 3,00 |
| Glycerin | 20,00 |
| Magnesiumsulfat 7 $H_2O$ | 0,80 |
| Parfüm, Konservierungsmittel, NaOH, Farbstoffe, Antioxidantien | q.s. |
| Wasser | ad 100,00 |

| Beispiel 8 (W/S-Emulsion): | |
|---|---|
| | Gew.-% |
| Laurylmethicone Copolyol | 2,50 |
| Dimethicone Dow Corning 200 Fluid, 5 cs | 10,00 |
| Dimethicone Wacker AK 10 | 6,00 |
| Dimethicone Th. Goldschmidt ABIL 20 | 3,00 |
| Dimethicone Wacker AK 50 | 1,00 |
| Glycerin | 20,00 |
| Natriumchlorid | 1,20 |
| Parfüm, Konservierungsmittel, NaOH, Farbstoffe, Antioxidantien | q.s. |
| Wasser | ad 100,00 |

| Beispiel 9 (Emulsions-Lippenpflegestift): | |
|---|---|
| | Gew.-% |
| Caprylic/Capric Triglyceride | 20,00 |
| Octyldodecanol | 20,00 |
| Dimethicone Dow Corning 200 Fluid, 10 cs | 5,00 |
| Dimethicone Dow Corning 200 Fluid, 20 cs | 5,00 |
| Polyglyceryl-3 Dioleat | 3,50 |
| Bienenwachs | 12,50 |
| Squalan | 11,00 |

(fortgesetzt)

| Beispiel 9 (Emulsions-Lippenpflegestift): | |
|---|---|
| | Gew.-% |
| $C_{20-40}$-Alkylstearate | 5,00 |
| Jojoba Öl | 10,00 |
| Carnauba Wachs | 2,00 |
| Tocopherolacetat | 0,75 |
| Parfüm, Konservierungsmittel, NaOH, Farbstoffe, Antioxidantien | q.s. |
| Wasser | ad 100,00 |

## Patentansprüche

**1.** Kosmetische oder dermatologische Zubereitungen, welche

(a) Wasser-in-Öl-Emulsionen darstellen und
(b) einen Gehalt von 3 bis zu 30 Gew.-%, bevorzugt 5 - 10 Gew.-% eines oder mehrerer Silicone der allgemeinen Struktur

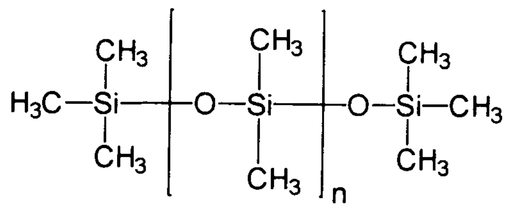

,

bezogen auf das Gesamtgewicht der Zubereitung, aufweisen, wobei n Zahlen zwischen 5 und 50 annehmen kann,
(c) höchstens 1 Gew.-% an cyclischen Siliconen aufweisen, bezogen auf das Gesamtgewicht der Zubereitung, wobei bevorzugt wird, vollständig auf solche Öle zu verzichten.
(d) höchstens 10 Gew.-%, bevorzugt 0,5 - 3 Gew.-% an Siliconen der allgemeinen Struktur

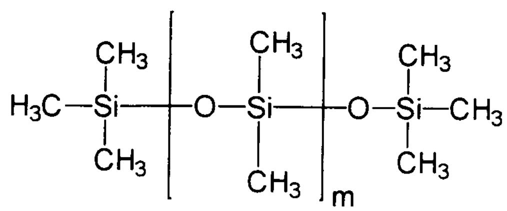

aufweisen, bezogen auf das Gesamtgewicht der Zubereitung, wobei m Zahlen größer als 50 annimmt.

**2.** Zubereitungen nach Anspruch 1, dadurch gekennzeichnet, daß der Durchschnittswert für n aus dem Bereich von 5 - 20, bevorzugt 12 -16, gewählt wird.

**3.** Zubereitungen nach Anspruch 1, dadurch gekennzeichnet, daß die Ölphase der Emulsionen im Sinne der vorliegenden Erfindung bis zur Hälfte des Gesamtgewichtes der Ölkomponenten aus der Gruppe der Ölkomponenten gewählt werden, die nicht aus Siliconölen bestehen.

Europäisches Patentamt

# EUROPÄISCHER RECHERCHENBERICHT

Nummer der Anmeldung
EP 99 11 7909

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | EP 0 281 394 A (RICHARDSON VICKS INC) 7. September 1988 (1988-09-07) * Beispiel 2 * | 1-3 | A61K7/48 |
| A | EP 0 661 042 A (SHISEIDO CO LTD) 5. Juli 1995 (1995-07-05) * Beispiel 12 * | 1-3 | |
| A | EP 0 331 833 A (SHISEIDO CO LTD) 13. September 1989 (1989-09-13) * Beispiele 3-15 * | 1-3 | |
| A | EP 0 456 459 A (UNILEVER PLC ;UNILEVER NV (NL)) 13. November 1991 (1991-11-13) * das ganze Dokument * | 1-3 | |

RECHERCHIERTE SACHGEBIETE (Int.Cl.7)

A61K

Der vorliegende Recherchenbericht wurde für alle Patentansprüche erstellt

| Recherchenort | Abschlußdatum der Recherche | Prüfer |
|---|---|---|
| DEN HAAG | 18. Februar 2000 | Couckuyt, P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 99 11 7909

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-02-2000

| Im Recherchenbericht angeführtes Patentdokument | Datum der Veröffentlichung | Mitglied(er) der Patentfamilie | Datum der Veröffentlichung |
|---|---|---|---|
| EP 0281394 A | 07-09-1988 | CA 1311194 A | 08-12-1992 |
| | | US 4960764 A | 02-10-1990 |
| EP 0661042 A | 05-07-1995 | JP 7233027 A | 05-09-1995 |
| | | US 5589165 A | 31-12-1996 |
| EP 0331833 A | 13-09-1989 | JP 1180237 A | 18-07-1989 |
| | | JP 2657504 B | 24-09-1997 |
| | | DE 3873313 A | 03-09-1992 |
| | | US 5015469 A | 14-05-1991 |
| EP 0456459 A | 13-11-1991 | AT 103170 T | 15-04-1994 |
| | | AU 636483 B | 29-04-1993 |
| | | AU 7640691 A | 14-11-1991 |
| | | CA 2041917 A | 11-11-1991 |
| | | DE 69101466 D | 28-04-1994 |
| | | DE 69101466 T | 14-07-1994 |
| | | DK 456459 T | 25-07-1994 |
| | | ES 2062683 T | 16-12-1994 |
| | | GB 2243780 A,B | 13-11-1991 |
| | | IN 172888 A | 25-12-1993 |
| | | JP 4226906 A | 17-08-1992 |
| | | JP 7045375 B | 17-05-1995 |
| | | US 5196187 A | 23-03-1993 |
| | | ZA 9103552 A | 27-01-1993 |

EPO FORM P0461

Für nähere Einzelheiten zu diesem Anhang : siehe Amtsblatt des Europäischen Patentamts, Nr.12/82